**Europäisches Patentamt**

**European Patent Office**

**Office européen des brevets**

(19)

(11) Numéro de publication: **0 222 672**
**B1**

(12) **FASCICULE DE BREVET EUROPEEN**

(45) Date de publication du fascicule du brevet: **25.07.90**

(51) Int. Cl.⁵: **A 61 G 1/00, A 61 B 6/04**

(21) Numéro de dépôt: **86420241.1**

(22) Date de dépôt: **25.09.86**

(54) **Dispositif d'immobilisation pour la réalisation d'actes médicaux et paramédicaux.**

(30) Priorité: **26.09.85 FR 8514496**
**01.10.85 FR 8514703**
**13.11.85 FR 8516972**
**08.01.86 FR 8600328**
**03.02.86 FR 8601595**

(43) Date de publication de la demande:
**20.05.87 Bulletin 87/21**

(45) Mention de la délivrance du brevet:
**25.07.90 Bulletin 90/30**

(84) Etats contractants désignés:
**AT BE CH DE FR GB IT LI LU NL SE**

(56) Documents cités:
**DE-A-1 803 349**    **US-A-3 650 523**
**DE-C- 824 668**    **US-A-3 655 178**
**FR-A-1 536 386**    **US-A-3 861 666**
**US-A-1 799 692**    **US-A-3 933 154**
**US-A-2 681 839**    **US-A-4 473 912**
**US-A-3 449 570**    **US-A-4 506 664**
**US-A-3 648 305**

(73) Titulaire: **Saussereau, Guy**
**37, Mont Salomon**
**F-38200 Vienne (FR)**

(72) Inventeur: **Saussereau, Guy**
**37, Mont Salomon**
**F-38200 Vienne (FR)**

(74) Mandataire: **Maureau, Philippe et al**
**Cabinet Germain & Maureau Le Britannia.- Tour**
**C 20, bld Eugène Déruelle Boîte Postale 3011**
**F-69392 Lyon Cédex 03 (FR)**

Courier Press, Leamington Spa, England.

## Description

La présente invention se rapporte à un dispositif d'immobilisation d'un patient, pour la réalisation sur celui-ci d'actes médicaux et paramédicaux, notamment d'actes radiologiques.

Pour pratiquer des actes radiologiques sur un enfant, une personne sénile ou une personne blessée, il est en général indispensable d'immobiliser le patient. Il existe à l'heure actuelle de nombreux dispositifs d'immobilisation comportant généralement un socle, plat ou en forme de gouttière, le patient étant maintenu sur ce socle par des bandes transversales ou par un dispositif analogue. Les documents US—A—3 655 178 et US—A—3 449 570 décrivent par exemple des dispositifs d'immobilisation de ce genre.

Ce dispositifs connus n'offrent pas la possibilité de maintenir efficacement de très jeunes enfants, de 0 à 3 ans environ, et de réaliser dans toutes les positions pratiquement tous les actes radiologiques sans avoir à détacher le patient. Ils sont par ailleurs construits pour une certaine taille de personne: bébés uniquement, enfants uniquement ou adultes uniquement, par exemple, et ne sont donc pas universels. Un grand laboratoire de radiologie doit donc posséder plusieurs de ces dispositifs d'immobilisation, quatre à cinq par exemple.

En ce qui concerne les personnes blessées, il n'existe pas à l'heure actuelle de dispositifs de contention aux parois rabattables sur les côtés du blessé, et qui permettent en fonction des soins, de l'immobiliser sur tout ou partie du corps, un tel dispositif permettant à la fois d'immobiliser le blessé sur le site, de le transporter à l'aide de ce dispositif de contention, et de pratiquer sur lui toutes sortes d'actes radiologiques à son arrivée à l'hôpital, sans avoir à le détacher et donc à le manipuler.

Le dispositif d'immobilisation de l'invention ne présente pas les inconvénients de ces dispositifs connus. Il est destiné à des personnes de toutes tailles, il maintient efficacement une personne sur le plan frontal, sur le plan dorsal, et sur les plans latéraux. Il permet de lui pratiquer, sans le détacher, des actes radiologiques dans toutes les positions et il peut servir à assurer l'immobilisation d'un blessé sur le site et son transport, sans qu'il soit nécessaire de le détacher à son arrivée à l'hôpital pour certains premiers soins et en tous cas pour les examens radiologiques. L'immobilisation est effectuée de manière rapide, sans exercer de pression ventrale, ce qui est très avantageux dans certains cas, et par des moyens simples et peu onéreux. Ce dispositif se présente sous la forme d'un appareil de contention ouvert sur le dessus et apte à recevoir le patient en position allongée. Il comporte un fond constitué par un socle rigide de forme oblongue, en matériau perméable aux rayons X, et des parois latérales rabattables contre le patient. Ces parois latérales sont en matériau rigide ou semi-rigide et perméable aux rayons X. Selon l'invention, elles ont une allure générale de gouttières, liées aux grands côtés du socle et dont la convexité est dirigée, en position rabattue contre le patient, vers l'axe longitudinal du socle, des organes de serrage perméables aux rayons X, par exemple des sangles, étant en outre prévus pour maintenir fermement ces gouttières en appui latéral contre le patient. De préférence, ce dispositif est entièrement constitué en matériaux amagnétiques, ce qui permet par exemple d'appliquer au patient des traitements par résonnance magnétique.

Le socle est préférentiellement plat et préférentiellement à contour rectangulaire. Il est avantageusement muni de poignées permettant d'utiliser le dispositif comme un brancard.

Ce dispositif est avantageusement apte à recevoir des accessoires de fixation ou suspension pour l'orienter dans différentes positions, horizontales ou verticales, afin de pratiquer divers actes radiologiques.

De toute façon, l'invention sera bien comprise, et ses avantages ainsi que d'autres caractéristiques ressortiront, au cours de la description suivante de quelques exemples de réalisation, en référence aux dessins schématiques annexés dans lesquels:

Figure 1 est une vue en perspective de ce dispositif dans une forme de réalisation très dépouillée,

Figure 2 est une vue en perspective de ce dispositif, en position ouverte sur un patient, dans une forme de réalisation un peu modifiée,

Figure 3 est une vue en bout du dispositif de la figure 2, avec interposition d'un matelas sous le patient,

Figure 4 est une vue similaire à la figure 3, avec les gouttières latérales rabattues, des coussins étant en outre interposés entre les gouttières et la tête du patient,

Figure 5 est une vue en perspective du même dispositif, en position refermée de maintien du patient,

Figures 6 à 9 représentent respectivement le socle, un gouttière latérale, une sangle de serrage, et le dispositif tout monté, dans une autre forme de réalisation.

Figure 10 est une variante de réalisation du dispositif de la figure 9,

Figures 11 à 14 représentent respectivement le socle, une gouttière latérale, une sangle de serrage, et le dispositif tout monté dans encore une autre forme de réalisation,

Figure 15 est une vue en perspective d'un dispositif d'immobilisation selon l'invention, équipé d'accessoires de suspension et d'orientation,

Figure 16 montre une potence de suspension de l'ensemble de la figure 15, à fixer sur une table de radiographie,

Figure 17 montre une potence de suspension du même ensemble à fixer contre un mur.

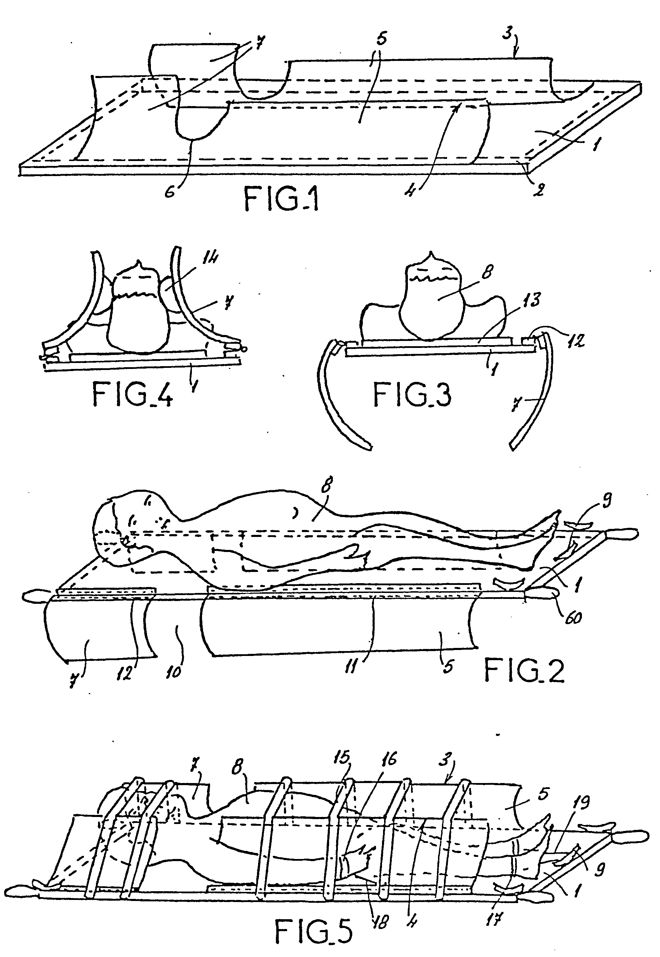

Dans la forme de réalisation de la figure 1, le dispositif d'immobilisation se compose d'un socle rigide, renforcé par un cadre 2 encore plus rigide, de forme rectangulaire, sur les grands côtés duquel sont soudés ou collés, tangentiellement à la surface supérieure du socle 1, les bords de deux gouttières semi-rigides 3, 4 formant les côtés du dispositif de contention. La convexité des gouttières 3, 4 est dirigée vers l'axe longitudinal du socle 1. Le socle 1, son cadre 2 et les gouttières 3, 4 sont en matériau plastique transparent, résistant mécaniquement, perméable aux rayons X et amagnétique. A titre d'exemple, on peut utiliser pour leur fabrication des polycarbonates.

Chaque gouttière latérale 3, 4 est composée, de droite à gauche sur le dessin, d'une première partie allongée 5 destinée au maintien du tronc et des membres inférieurs, d'une partie échancrée 6, pour le passage éventuel des bras, et d'une dernière partie 7, beaucoup plus courte et légèrement plus large que la partie 5, destinée au maintien de la tête. La plus grande largeur de la partie 7 a surtout pour but d'éviter les images parasites.

En utilisation, on allonge le patient sur le socle 1, on rabat, en appui sur les côtés du patient, les parois latérales 3 et 4, et l'on serre celles-ci contre ce patient à l'aide de sangles, perméables aux rayons X, non représentées sur cette figure. Les gouttières latérales semi-rigides emprisonnent alors le malade latéralement tout en l'appuyant légèrement en direction du socle.

La figure 2 montre une variante de ce dispositif de contention, avec un patient 8 allongé sur le socle 1. Le socle 1 est muni de poignées 60, qui en font aussi un brancard, de sorte que le dispositif de contention peut aussi servir au transport du patient. Des crochets 9 sont rajoutés le long des petits côtés du socle, afin d'y fixer des lacets de tension de bracelets ou chevillières. Les parties 5 et 7 d'une même gouttière, qui sont ici séparées par un espace 10 pour laisser passer les bras, sont articulés sur les bords longitudinaux du socle au moyen de charnières 11, 12, qui sont fixées sur les gouttières 5, 7 d'une part et sur le socle 1 d'autre part.

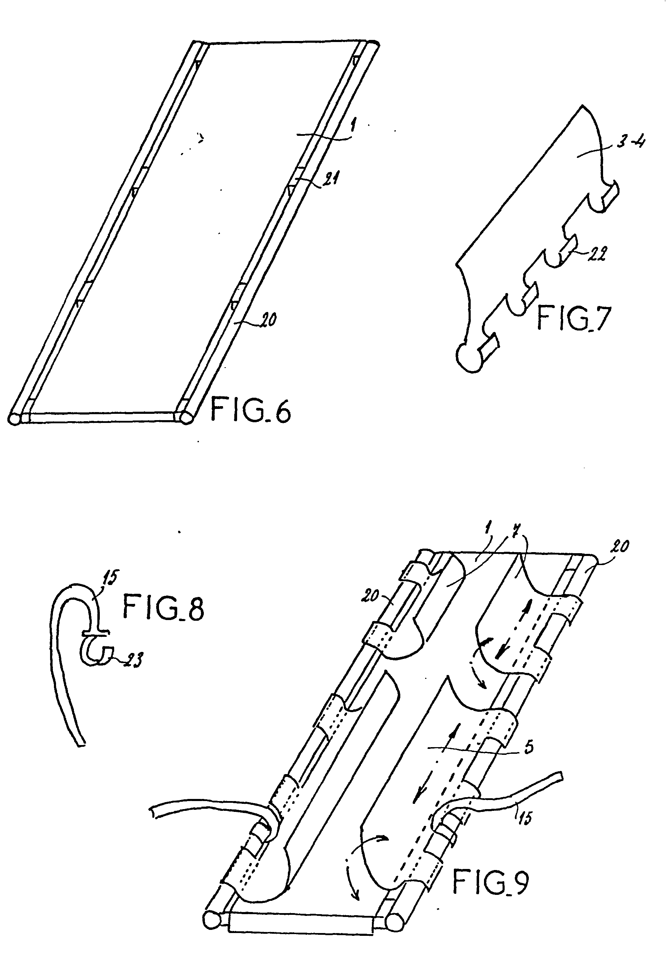

La figure 3 montre qu'il est possible de placer, entre le malade 8 et le socle 1, un matelas 13 constitué, selon un aspect de l'invention, d'une mousse plastique à cellules fermées. L'inventeur a trouvé que ce matériau présente d'excellentes qualités mécaniques et radiologiques. Cette mousse plastique est par exemple un polyuréthane. Le matelas 13 améliore le confort et permet d'éviter les images parasites.

La figure 4 montre qu'il est possible, lorsqu'on rabat les gouttières latérales 7 vers le tête du patient, d'interposer en plus des coussins 14 entre ces gouttières et la tête. Préférentiellement, ces coussins sont aussi en une mousse plastique à cellules fermées.

La figure 5 représente le patient 8 immobilisé grâce au dispositif de la figure 2. L'ensemble des gouttières 7, 5, est rabattu vers le patient, ces gouttières appuyant non seulement latéralement mais aussi de haut en bas, en biais et en direction de l'axe du socle, sans exercer de pressions ventrale et frontale. Des sangles 15, en matériau perméable aux rayons X et amagnétique, passent autour de l'ensemble et constituent, une fois tendues et leurs deux extrémités fixées l'une à l'autre par nouage ou autre moyen, des organes de serrage qui tendent à rapprocher l'un vers l'autre les bords libres des gouttières latérales associées 3, 4 de part et d'autre du socle 1. Des bracelets 16 et des chevillières 17 sont passés autour des poignets et des chevilles du patient, et des lacets 18, 19 les relient aux crochets 9. Les lacets 18, 19 sont fortement tendus, afin de maintenir les bras et les jambes du patient en hyperextension.

Une autre forme de réalisation de ce dispositif de contention, qui a l'avantage de le rendre entièrement et rapidement démontable, est représentée aux figures 6 à 9.

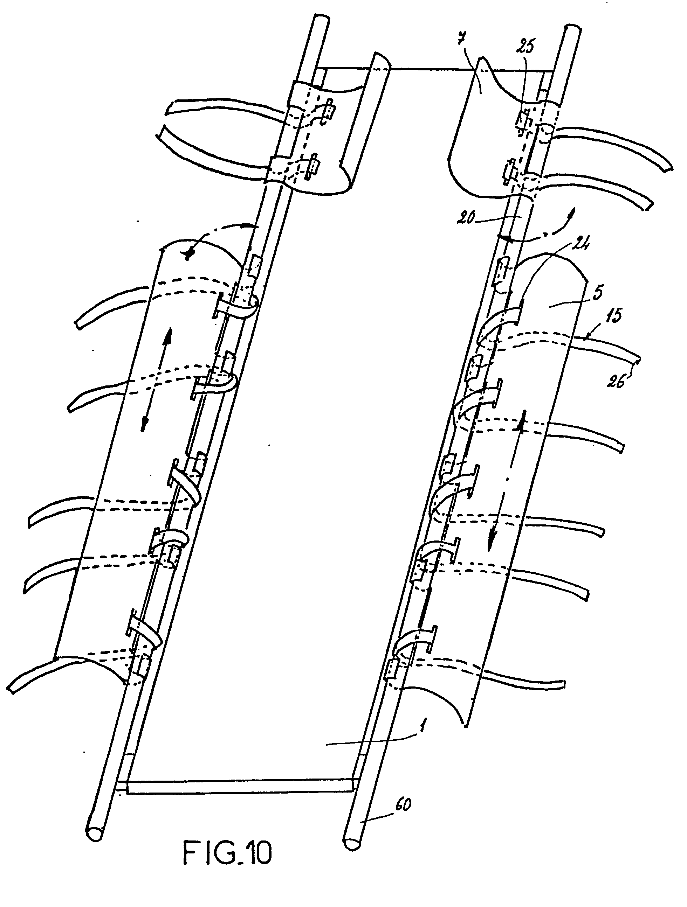

Selon cette réalisation, le socle est bordé sur ses grands côtés par des barres cylindriques 20, légèrement écartées des bords du socle par l'interposition d'entretoises 21 (figure 6). Les gouttières 3, 4 (figure 7) sont toutes munies, du côté qui est articulé sur les bords du socle, d'attaches rapides constituées par des anneaux ouverts 22 de fixation autour des barres 20. Ces anneaux ouverts 22 sont de convexité opposée à celle de la gouttière 5, cette gouttière 5 et chaque anneau 22 étant réunis par leur génératrice. Pareillement, les sangles de fixation 15 (figure 8) sont aussi munies d'anneaux ouverts 23 semi-rigides de fixation autour des barres 20.

L'appareil tout monté, après enclenchement autour des barres latérales des anneaux 22, 23 est représenté à la figure 9. Préférentiellement, les entretoises 21 sont placées, relativement aux anneaux ouverts 22, de façon à autoriser un certain glissement de chaque gouttière 5, 7 (figure 9) le long de la barre 20 sur laquelle elle est articulée: on peut alors ajuster la position de chaque gouttière le long de la bordure longitudinale du socle 1.

Les extrémités libres des sangles 15 sont garnies par exemple de fermetures auto-agrippantes, du genre VELCRO (marque déposée), comme il est d'ailleurs également possible pour les sangles du dispositif de la figure 5.

La figure 10 représente une variante de réalisation du dispositif de la figure 9. Cette réalisation diffère de la précédente en ce que les barres cylindriques latérales sont prolongées de 10 à 20 cm à chacune de leurs extrémités, ce qui définit quatre poignées 60 permettant d'utiliser le socle 1 comme un brancard. Par ailleurs, les gouttières latérales articulées 5, 7 sont percées, près de leur articulation, d'une série de fentes alignées 24, dans chacune desquelles on introduit une sangle 15. Dans ce cas, ces sangles 15 ne comportent pas d'anneaux ouverts, mais en leurs lieux et place, elles comportent, à l'extrémité opposée à leur extrémité libre, un bourrelet d'arrêt en fin de course 25, constitué par exemple par une surépaisseur de tissu. On introduit dans chaque fente 24, du côté concave de la gouttière 5, l'extrémité libre 26 d'une sangle 15; on la fait ensuite passer entre le cylindre 20 voisin et le socle 1, pour contourner ce cylindre 20 et la face concave de la gouttière 5. En faisant passer les extrémités 26 au-dessus du socle 1, on fait basculer vers le patient la gouttière 5, et l'on serre l'ensemble en attachant les extrémités 26 des sangles 15 aux extrémités des sangles homologues situées symétriquement de l'autre côté du socle 1. Cette forme de réalisation est avantageuse pour l'immobilisation du patient, car les sangles 15 tendent à la fois, en tirant sur la partie basse de chaque gouttière, à rapprocher cette partie basse des flancs du patient, et en poussant sur la tranche supérieure de chaque gouttière en direction de l'axe longitudinal du socle, à bien exercer sur le patient la pression de haut en bas l'appuyant sur le socle.

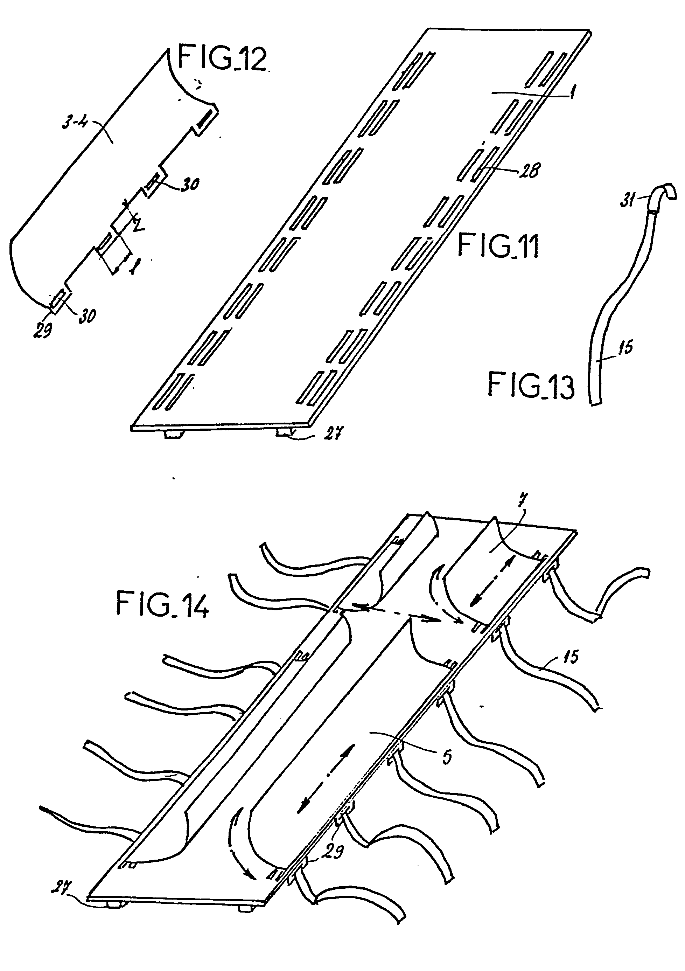

Les figures 11 à 14 sont les homologues des figures 6 à 9 dans encore une autre forme de réalisation. Le socle 1, qui est ici renforcé par des longerons 27 placés sous le socle, est garni, sur chacun de ses grands côtés, de deux rangées de fentes 28, ces rangées étant parallèles à l'axe longitudinal du socle et parallèles entre elles.

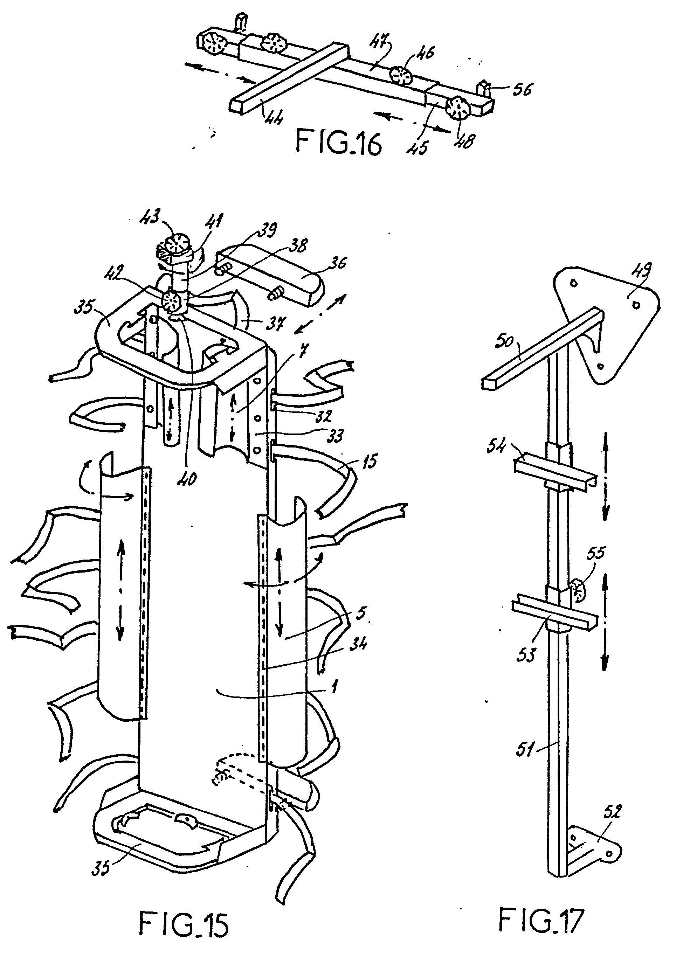

Les gouttières 3, 4 sont garnies du côté qui vient s'articuler sur le socle 1, de pattes 29, de largeur 1 inférieure à celle des fentes 28, chaque patte 29 étant également percée d'une fente 30 parallèle à l'axe longitudinal de la gouttière. La longueur L de chaque patte 29 est largement supérieure à l'épaisseur du socle 1, afin qu'elles dépassent, avec leur fente 30, sous celui-ci (voir figure 14).

Les sangles 15 sont chacune garnies à une de leurs extrémités d'un crochet 31 qui permet de les fixer, après mise en place des gouttières par introduction des pattes 29 dans une des deux rangées de fentes 28, dans une des fentes 30. Un autre moyen d'arrêt, tel qu'une surépaisseur de tissu, pourrait être utilisé à la place du crochet 31.

Le montage s'effectue comme suit (figure 14):

Selon la largeur du patient, on introduit les pattes 29 des gouttières 5, 7 dans l'une ou l'autre des deux rangées parallèles de fentes 28: pour un patient mince, on les introduit dans la rangée la plus proche de l'axe longitudinal, et pour un patient plus fort on les introduit dans la rangée la plus proche du bord latéral du socle 1. On ajuste alors, en les déplaçant longitudinalement dans les fentes 28, les positions axiales des gouttières 5, 7. L'immobilisation s'effectue ensuite en accrochant les sangles 15 dans les fentes 30, et en les tendant au-dessus du patient pour les attacher finalement aux sangles homologues, comme précédemment.

Selon un autre aspect de l'invention, le dispositif de contention est apte à recevoir des accessoires destinés à l'orienter à loisir pour pouvoir procéder sur le patient à toutes sortes d'actes radiologiques. La figure 15 montre un dispositif de contention, d'ailleurs dans encore une autre forme de réalisation, qui est équipé de tels accessoires d'orientation.

Dans le mode de réalisation de la figure 15, les sangles 15 sont introduites dans des fentes latérales 32 réalisées en bordure des grands côtés du cadre 1 ou dans son cadre de renfort. Elles font le tour du socle, ou sont arrêtées comme précédemment (Cf. figure 10) en fin de course à l'entrée des fentes 32 par une butée ou une surépaisseur de tissu. Par ailleurs, toutes les gouttières latérales 5, 7 sont ajustables en position longitudinale par coulissement dans des glissières parallèles à l'axe longitudinal du socle: dans cet exemple, les gouttières latérales 7 qui maintiennent la tête sont pressées, avec possibilité de glissement longitudinal, entre des plaques 33 et le socle, tandis que les gouttières 5 qui maintiennent le tronc et les jambes sont fixées sur des charnières qui coulissent elles-mêmes longitudinalement dans des glissières, non représentées mais semblables aux glissières 33.

Sur les petits côtés du socle 1 sont rapportés, par vissage ou éventuellement soudage, deux cadres d'orientation 35. Les cadres 35 sont perpendiculaires au socle 1 et situés du côté apte à recevoir le patient. Ils ont une forme de rectangle à coins supérieurs arrondis. Ils peuvent avoir une autre forme, par exemple semi-circulaire. Ils sont chacun prolongés, de l'autre côté du socle, par une pièce amovible 36, sensiblement de même contour, mais moins haute par rapport à la surface du socle 1.

Sous le socle 1, à son extrémité recevant la tête du patient, est fixée une plaque métallique 37 sur laquelle est soudée un tube creux 38, en acier par exemple, d'axe parallèle à celui du socle 1. Dans le tube cylindrique creux 38 peut tourillonner une tige circulaire 39, arrêtée à son extrémité inférieure par un collerette 40, et comportant à sa partie supérieure une portion de tube à section rectangulaire 41, d'axe perpendiculaire à celui de la tige 39. Les tubes creux 38 et 41 sont traversés par des vis de blocage 42, 43 le tube 41 est destiné à recevoir un tige, carrée ou rectangulaire, servant de potence de suspension ou de bras de fixation.

La figure 16 montre un exemple de dispositif pour relier l'ensemble de la figure 15 à une table de radiologie. Il se compose d'une tige 44, de section rectangulaire apte à permettre son coulissement étroit dans le petit tube 41, et qui est soudée orthogonalement sur une barre de fixation 47, extensible de manière télescopique grâce à des éléments coulissants 45, blocables à la bonne longueur par des vis 46. La barre 47 est apte à se fixer par des liaisons 56 sur un profilé de table de radiologie, des vis 48 servant à la bloquer en position sur ce profilé.

Ce dispositif d'orientation permet de placer le patient dans toutes les positions: de face, de profil, en oblique, debout, couché, sur le ventre ou sur le dos. L'orientation du patient est effectuée en faisant tourner le socle 1 autour de l'axe du cylindre 39.

La figure 17 montre un dispositif remplaçant celui de la figure 16 dans le cas où ne dispose pas d'une table de radiologie. Il comporte une plaque supérieure 49 de fixation de l'ensemble par exemple contre un mur, une barre horizontale rectangulaire 50 formant potence à l'instar de la barre 44 de la figure 16, une barre maîtresse 51 verticale et positionnée à distance du mur, et une plaque inférieure 52 de fixation au mur.

Le long de la barre verticale 51 coulissent deux mâchoires horizontales 53, 54 porte-cassettes radiographiques connues en soi. La mâchoire inférieure 53 est blocable en position sur la barre 51 au moyen d'une vis 55.

L'invention n'est bien entendu pas limitée aux exemples de réalisation qui viennent d'être décrits, et de nombreuses variantes sont imaginables. La courbure des gouttières latérales peut ne pas être régulière; elles peuvent être constituées de positions de courbes, ou de segments de droites, raccordés entre eux. Les gouttières latérales peuvent être rigides, au lieu de semi-rigides comme c'est préférentiellement le cas: on interposera alors, la plupart du temps, des coussins entre les gouttières et le patient. Les modes de fixation des gouttières latérales sur le socle peuvent être autres que ceux décrits: moulage, rivetage, vissage. L'invention n'est pas limitée à l'application aux être humains, et peut avantageusement être appliquée au domaine vétérinaire. Un seul modèle, adapté aux animaux à quatre pattes, de cet appareil d'immobilisation peut par exemple être utilisé dans une clinique vétérinaire pour les examens radiologiques, ou par résonnance magnétique, des chats et des chiens de petite taille ou de taille moyenne. Certaines charnières, ou éléments résistants de ce type peuvent être métalliques, si elles sont situées en des points peu gênants du point de vue radiologique. Les sangles peuvent être des sangles à boucles. Le socle, bien que préférentiellement plat, peut être dans certains cas de forme légèrement bombée, avec convexité ou concavité dirigée vers le patient. Le socle peut ne pas être rectangulaire. Les gouttières peuvent n'être agencée que pour maintenir une partie du corps du patient par exemple la tête, etc.

## Revendications

1. Dispositif d'immobilisation d'un patient en vue de lui pratiquer des actes médicaux et paramédicaux, notamment radiologiques, du type comportant un fond constitué par un socle rigide (1) de forme oblongue en matériau perméable aux rayons X apte à recevoir le patient (8) en position allongée et des parois latérales (3, 4) rigides en matériau perméable aux rayons X, caractérisé en ce que ces parois latérales (3, 4) sont liées aux grands côtés du socle (1) et rabattables contre le patient, ces parois (3, 4) ayant en outre une allure générale de gouttières, à convexité dirigée, en position rabattue contre le patient, vers l'axe longitudinal du socle (1), des organes de serrage (15) perméables aux rayons X étant prévus pour maintenir fermement ces gouttières latérales (3, 4) en appui latéral contre le patient.

2. Dispositif d'immobilisation selon la revendication 1, caractérisé en ce qu'il est réalisé en matériaux amagnétiques.

3. Dispositif d'immobilisation selon l'une des revendications 1 ou 2, caractérisé en ce que ces gouttières latérales rabattables (3, 4) sont en une matière semi-rigide.

4. Dispositif selon la revendication 3, caractérisé en ce que ces gouttières latérales (3, 4) sont fixées sur les grands côtés du socle (1), tangentiellement à sa surface supérieure.

5. Dispositif selon l'une des revendications 1 ou 2, caractérisé en ce que ces gouttières latérales rabattables (11) sont articulées sur les bords du socle.

6. Dispositif selon la revendication 5, caractérisé en ce qu'il est équipé de moyens (20—22, 28—29, 33—34) pour ajuster la position longitudinale des gouttières latérales rabattables (3—7).

7. Dispositif selon l'une des revendications 1 à 6, caractérisé en ce que le socle (1) est équipé de poignées (60) pour l'utiliser en brancard.

8. Dispositif selon l'une des revendications 1 à 7, caractérisé en ce que le socle (1) est plat.

9. Dispositif selon l'une des revendications 1 à 8, caractérisé en ce que le socle (1) est à contour rectangulaire.

10. Dispositif selon l'une des revendications 1 à 9, caractérisé en ce que le socle (1) est renforcé par un cadre (2).

11. Dispositif selon l'une des revendications 1 à 9, caractérisé en ce que le socle (1) est renforcé par les longerons (27).

12. Dispositif selon l'une des revendications 1 à 11, caractérisé en ce que chaque gouttière latérale (3 ou 4) est composée d'au moins deux gouttières (5, 7) séparées par un espace (10), une de ces gouttières (7) étant destinée à maintenir la tête du patient.

13. Dispositif selon la revendication 12, caractérisé en ce que la gouttière (7), apte à maintenir la tête du patient, est plus haute que les autres (5).

14. Dispositif selon l'une des revendications 1 à 13, caractérisé en ce qu'il est apte à recevoir un matelas (13) et des coussins (14), et en ce que ces matelas et ces coussins sont en une mousse plastique à cellules fermées.

15. Dispositif selon l'une des revendications 5 à 14, caractérisé en ce que les gouttières (5, 7) sont démontables et fixées sur les bords du socle par des attaches rapides (22, 29).

16. Dispositif selon la revendication 15, caractérisé en ce qu'il est équipé de moyens (28, 29) pour changer l'éloignement des gouttières (3, 4) par rapport à l'axe longitudinal du socle.

17. Dispositif selon l'une des revendications 1 à 16, caractérisé en ce que les gouttières sont munies de fentes (24) de passage des sangles (15), ces fentes (24) étant situées près du bord lié au socle, et les sangles (15) étant passées de façon que leur serrage entraîne, en position d'immobilisation du patient, une traction de la partie basse de ces gouttières en direction de celui-ci.

18. Dispositif selon l'une des revendications 1 à 17, caractérisé en ce que les gouttières latérales (3, 4) sont agencées pour ne maintenir qu'une partie du corps du patient.

19. Dispositif selon l'une des revendications 1 à 18, caractérisé en ce qu'il est apte à recevoir des accessoires (37 à 56) de fixation ou suspension pour l'orienter dans différentes positions.

20. Dispositif selon l'une des revendications 1 à 19, caractérisé en ce qu'au moins ses parois rabattables (3, 4) sont en un matériau transparent.

## Patentansprüche

1. Vorrichtung zur Immobilisierung eines Patienten zur Verabreichung medizinischer oder paramedizinischer Hilfe, insbesondere für die Durchführung von Röntgenaufnahmen, vom Typ bestehend aus einem Fond, gebildet durch einen steifen Unterbau länglicher Form (1) aus röntgenstrahlendurchlässigem Material, geeignet, den Patienten (8) in liegender Position aufzunehmen, und durch steife Seitenteile (3, 4) aus röntgenstrahlendurchlässigem Material, dadurch gekennzeichnet, daß die Seitenteile (3, 4) an den Längsseiten des Unterbaus (1) verbunden und gegen den Patienten klappbar sind, wobei diese Seitenteile (3, 4) desweiteren von gewölbtem Aussehen sind, mit steifer Krümmung, und bei zum Patienten geklappter Position, zur Längsachse des Unterbaus (1) zeigend, wobei röntgenstrahlendurchlässige Spannelemente (15) vorgesehen sind, um die gewölbten Seitenteile (3, 4) in seitlicher Lage gegen den Patienten zu halten.

2. Vorrichtung zur Immobilisierung nach Patentanspruch 1, dadurch gekennzeichnet, daß sie aus unmagnetischem Material hergestellt ist.

3. Vorrichtung zur Immobilisierung nach dem Patentanspruch 1 oder 2, dadurch gekennzeichnet, daß die klappbaren gewölbten Seitenteile (3, 4) aus halbsteifem Material bestehen.

4. Vorrichtung nach Patentanspruch 3, dadurch gekennzeichnet, daß die gewölbten Seitenteile (3, 4) an den Längsseiten des Unterbaus (1) tangentiell zur Oberfläche befestigt sind.

5. Vorrichtung nach dem Patentanspruch 1 oder 2, dadurch gekennzeichnet, daß die klappbaren gewölbten Seitenteile (11) an den Kanten des Unterbaus beweglich sind.

6. Vorrichtung nach Patentanspruch 5, dadurch gekennzeichnet, daß sie mit Mitteln (20—22, 28—29, 33—34) ausgerüstet ist, um die Längsposition der klappbaren gewölbten Seitenteile (3—7) einzustellen.

7. Vorrichtung nach einem der Patentansprüche 1 bis 6, dadurch gekennzeichnet, daß der Unterbau (1) mit Griffen (60) versehen ist, um die Vorrichtung als Tragbare zu verwenden.

8. Vorrichtung nach einem der Patentansprüche 1 bis 7, dadurch gekennzeichnet, daß der Unterbau (1) flach ist.

9. Vorrichtung nach einem der Patentansprüche 1 bis 8, dadurch gekennzeichnet, daß der Unterbau (1) von rechteckiger Form ist.

10. Vorrichtung nach einem der Patentansprüche 1 bis 9, dadurch gekennzeichnet, daß der Unterbau (1) durch einen Rahmen (2) verstärkt ist.

11. Vorrichtung nach einem der Patentansprüche 1 bis 9, dadurch gekennzeichnet, daß der Unterbau (1) mit Streben (27) verstärkt ist.

12. Vorrichtung nach einem der Patentansprüche 1 bis 11, dadurch gekennzeichnet, daß jedes gewölbte Seitenteil (3 und 4) aus mindestens zwei Seitenteilen (5, 7) besteht, die durch einen freien Raum (10) abgetrennt werden, wobei eins dieser gewölbten Seitenteile (7) für den Halt des Kopfes des Patienten bestimmt ist.

13. Vorrichtung nach Patentanspruch 12, dadurch gekennzeichnet, daß das gewölbte Seitenteil (7), das den Kopf des Patienten hält, höher als die andern (5) ist.

14. Vorrichtung nach einem der Patentansprüche 1 bis 13, dadurch gekennzeichnet, daß eine Matratze (13) und Kissen (14) untergeschoben werden können, und daß diese Matratze und diese Kissen aus Schaumstoff mit geschlossenen Zellen bestehen.

15. Vorrichtung nach einem der Patentansprüche 5 bis 14, dadurch gekennzeichnet, daß die gewölbten Seitenteile (5, 7) demontierbar und an den Kanten des Unterbaus mit Schnellbefestigungen (22, 29) montiert sind.

16. Vorrichtung nach Patentanspruch 15, dadurch gekennzeichnet, daß sie mit Mitteln (28, 29) ausgerüstet ist, mit denen der Abstand der gewölbten Seitenteile (3, 4) zur Längsachse des Unterbaus (1) geändert werden kann.

17. Vorrichtung nach einem der Patentansprüche 1 bis 16, dadurch gekennzeichnet, daß die gewölbten Seitenteile mit Schlitzen (24) versehen sind, durch die die Gurte (15) gezogen werden, wobei diese Schlitze (24) dicht am Rand angebracht sind, der mit dem Unterbau verbunden ist und die Gurte (15) so durchgeführt sind, daß sie beim Anspannen, in der Immobilisierungsposition des Patienten, eine Zugspannung auf die untere Partie der gewölbten Seitenteile in Richtung des Patienten ausüben.

18. Vorrichtung nach einem der Patentansprüche 1 bis 17, dadurch gekennzeichnet, daß die gewölbten Seitenteile (3, 4) so angeordnet sind, daß sie den Körper eines Patienten halten.

19. Vorrichtung nach einem der Patentansprüche 1 bis 18, dadurch gekennzeichnet, daß sie mit Befestigungszubehör oder Aufhängezubehör (37 bis 56) ausgerüstet werden kann zur Orientierung des Patienten in verschiedene Positionen.

20. Vorrichtung nach einem der Patentansprüche 1 bis 19, dadurch gekennzeichnet, daß mindestens eine der klappbaren Seitenteile (3, 4) aus durchsichtigem Material besteht.

## Claims

1. Device for immobilizing a patient for medical and paramedical treatment, particularly radiological treatment, of the type consisting of an oblong rigid base (1) made from a material allowing passage of X-rays, capable of holding a patient (8) in the lying position and having rigid side sections (3, 4) allowing passage of X-rays, characterized by the fact that these side sections are linked to the long sides of base (1) and may be folded up against the patient, these side sections (3, 4) also having the general appearance of channel sections, and their convex faces, when folded up against the patient, being towards the longitudinal axis of base (1), tightening devices (15) allowing passage of X-rays being designed to hold these side sections (3, 4) firmly against the patient's sides.

2. Immobilizing device according to claim 1, characterized by the fact that it is made of nonmagnetic materials.

3. Immobilizing device according to claims 1 or 2, characterized by the fact that these fold-up side sections (3, 4) are made of semi-rigid material.

4. Device according to claim 3, characterized by the fact that these side sections (3, 4) are attached to the long sides of base (1), tangentially to its upper surface.

5. Device according either to claim 1 or 2, characterized by the fact that these fold-up side sections (11) are hinged to the edges of the base.

6. Device according to claim 5, characterized by the fact that it is equipped with means (20—22, 28—29, 33—34) for longitudinal position adjustment of the fold-up side sections (3—7).

7. Device according to one of claims 1 to 6, characterized by the fact that base 1 is fitted with handles (60) for use as a stretcher.

8. Device according to one of claims 1 to 7, characterized by the fact that base (1) is flat.

9. Device according to one of claims 1 to 8, characterized by the fact that base (1) has a rectangular shape.

10. Device according to one of claims 1 to 9, characterized by the fact that base (1) is reinforced by a frame (2).

11. Device according to one of claims 1 to 9, characterized by the fact that base (1) is reinforced by spars (27).

12. Device according to one of claims 1 to 11, characterized by the fact that each side (3 or 4) consists of at least two side sections (5, 7) separated by a space (10), one of these side sections (7) being designed to hold the patient's head.

13. Device according to claim 12, characterized by the fact that side sections (7) designed for holding the patient's head are higher than the others (5).

14. Device according to one of claims 1 to 13, characterized by the fact that it may be fitted with a mattress (13) and cushions (14), and by the fact that these mattresses and cushions are made from a closed-cell plastic foam.

15. Device according to one of claims 5 to 14, characterized by the fact that the side sections (5, 7) are removable and attached to the edges of the base by quick-release attachments (22, 29).

16. Device according to claim 15, characterized by the fact that it is equipped with means (28, 29) for changing the distance of side sections (3, 4) from the longitudinal axis of the base.

17. Device according to one of claims 1 to 16, characterized by the fact that the side sections have belt-slots (24) through which belts (15) can be passed, these slots (24) being located near the edge nearest to the base, and belts (15) being fitted in such a way that, when tightened in immobilizing position, the lower part of these side sections is drawn in towards the patient.

18. Device according to one of claims 1 to 17, characterized by the fact that the side sections (3, 4) are configured in such a way as to hold only one part of the patient's body.

19. Device according to one of claims 1 to 18, characterized by the fact that it can be fitted with attachment or mounting accessories (37 to 56), for orientation in different positions.

20. Device according to one of claims 1 to 19, characterized by the fact that at least its fold-up side sections (3, 4) are made of a transparent material.

FIG.1

FIG.4

FIG.3

FIG.2

FIG.5

1

FIG. 6

FIG. 7

FIG. 8

FIG. 9

FIG.10

FIG_12

3-4

30

29

30

FIG_11

1

28

31

15

FIG_13

FIG_14

7

15

5

29

27

FIG.16

FIG.15

FIG.17